# EUROPEAN PATENT APPLICATION

(11) **EP 4 595 771 A1**
(43) Date of publication of application: **06.08.2025**
(21) Application number: 23898334.0
(22) Date of filing: 30.11.2023
(51) Int. Cl.: A23L 5/20, A23L 29/00, A23L 3/46, A23P 10/40, A23L 5/00, C12P 13/06, C12P 13/10, C12P 13/08, C12M 1/00

(54) **FERMENTED PRODUCT PRODUCTION PROCESS AND PRODUCTION DEVICE**

(30) Priority: 30.11.2022 KR 20220164715
(71) Applicant: CJ CheilJedang Corporation, Seoul 04560 (KR)
(72) Inventor: HONG, Je-won, Seoul 04560 (KR); PARK, Shin-ae, Seoul 04560 (KR); KIM, Jungwon, Seoul 04560 (KR); YOO, Hyun-Woo, Seoul 04560 (KR); YU, Jaehun, Seoul 04560 (KR)
(74) Representative: Meissner Bolte Partnerschaft mbB
(86) International application number: PCT/KR2023/019550
(87) International publication number: WO 2024/117810

(57) **Abstract**

Provided is a method of producing a fermentation product, the method comprising a first step of preparing a deionized process liquid by removing ions from a process liquid comprising the fermentation product through capacitive deionization (CDI); and a second step of spray-drying the deionized process liquid to obtain the fermentation product without re-crystallization of the fermentation product. According to the present disclosure, since an ion exchange resin tower is not used, the fermentation product may be produced in an environmentally friendly manner, and since the reduction in the concentration of the fermentation product in the process liquid due to dilution during the ion removal process may be avoided, a high-purity fermentation product may be obtained with a high yield.

## Description

### [Technical Field]

The present disclosure relates to a production method and device for obtaining a high-purity fermentation product with a high yield in an environmentally friendly manner.

### [Background Art]

Capacitive deionization (CDI) is a technology that removes ions by adsorbing ions onto an electrode with a high specific surface area using electrochemical principles. When power is applied to the electrode surface, ions are adsorbed, and when adsorption reaches saturation, ions can no longer be adsorbed, and thus a reverse potential is applied to desorb the ions, regenerating the electrode. Since CDI and MCDI technologies operate at low potentials, they consume low energy. Since CDI and MCDI technologies do not use acids, bases, or salts when regenerating electrodes, they are considered environmentally friendly technologies.

Patents related to CDI or MCDI include electrode technology, module technology, and application technology, and among them, the application technology of CDI and MCDI are mainly used in water softeners and water desalination devices. 'KR 10-2022-0096174 A' describes an electrodeionization-type water purifier, in which an electrodeionization-type filter and module are used to purify water. 'KR 10-2333880 B1' describes a device and method for continuously supplying purified water using a CDI water purifier. In 'KR-10-1949140', ions are separated using CDI, and then the separated ions are solidified, and the solidified ions are used in manufacturing mineral beverages.

Such patents are mainly about purifying water or collecting ions to utilize ions.

### [Disclosure]

### [Technical Problem]

When fermentation products of amino acids, peptides, and polyphenols are produced from a fermentation broth, ions in the products are problematic, and in the present disclosure, it is confirmed that CDI may be fully used in this case as well, and it is intended to apply CDI in the production of food fermentation products or food material products from a fermentation broth.

### [Technical Solution]

An object of the present disclosure is to provide a process capable of removing ions in an environmentally friendly manner without using ion exchange resins via a CDI process and thereby producing high-purity fermentation products with high yields.

Further, another object of the present disclosure is to reduce process costs in a process of producing fermentation products.

### [Advantageous Effects]

When a method and device for producing a fermentation product according to one aspect of the present disclosure are used, the fermentation product may be produced in an environmentally friendly manner using a CDI process without an ion exchange resin.

Further, according to the present disclosure, since the fermentation product is not diluted by an eluting solution, the energy required for drying may be reduced.

Further, according to the present disclosure, since the fermentation product is produced using a spray-drying process without crystallization after the deionization process, the generation of a mother liquor and the loss of the fermentation product may be reduced, thereby increasing the process yield.

### [Brief Description of the Drawing]

FIG. 1 is a flow chart showing a method of producing a fermentation product according to the present disclosure;
FIG. 2 is a block diagram showing a device for producing a fermentation product according to the present disclosure;
FIG. 3 is a block diagram showing a CDI device within the device for producing a fermentation product according to one embodiment of the present disclosure;
FIG. 4 is a flow chart showing a method of producing a fermentation product according to the present disclosure; and
FIG. 5 is a graph analyzing the particle size of a fermentation product produced according to one embodiment of the present disclosure.

### [Detailed Description of Preferred Embodiments]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below.

The present disclosure relates to a technology for producing a fermentation product via a CDI process and a spray-drying. Since an ion exchange resin is not used during the production process, the technology is environmentally friendly, and since the fermentation product is not diluted during the process, the cost associated with steam drying of the fermentation product may be reduced. In addition, since the fermentation product is produced through the spray-drying process after ion removal, a re-crystallization process after ion removal is not necessary, and therefore, the amount of mother liquor generated may be reduced, thereby improving the yield efficiency of the fermentation product.

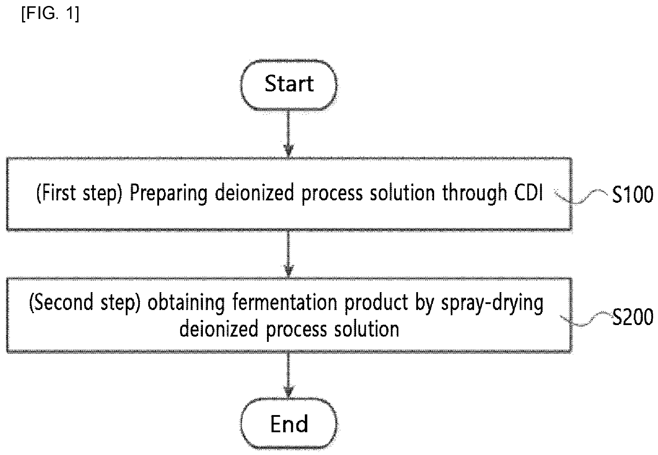

FIG. 1 is a flow chart showing the method of producing the fermentation product according to the present disclosure.

Referring to FIG. 1, a method of producing a fermentation product according to the present disclosure comprises a first step (S100) of preparing a deionized process liquid by removing ions from a process liquid comprising the fermentation product via capacitive deionization (CDI); and a second step (S200) of spray-drying the deionized process liquid to obtain the fermentation product without re-crystallization of the fermentation product. Hereinbelow, each step will be described in detail.

The first step (S100) is a step of preparing the process liquid comprising the fermentation product, and preparing the deionized process liquid by removing ions in the process liquid via capacitive deionization (CDI).

In the first step (S100), the process liquid may be prepared by fermentation utilizing microbial cells. The fermentation product comprised in the process liquid may be amino acids, peptides, or polyphenols.

For example, when the fermentation product is an amino acid, the amino acid may comprise at least one selected from the group consisting of glycine, alanine, serine, proline, valine, threonine, cysteine, isoleucine, leucine, asparagine, aspartic acid, glutamine, lysine, glutamic acid, methionine, histidine, phenylalanine, selenocysteine, arginine, tyrosine, citrulline, ornithine, and tryptophan. The fermentation product that may be produced according to the present disclosure may be any substance as long as it is not adsorbed together with ions during the CDI process. In some cases, the fermentation product comprised in the process liquid may be at least one selected from the group consisting of leucine, citrulline, isoleucine, and valine.

The process liquid prepared in the first step (S100) may be a fermented material produced by a strain. As used herein, the term "fermented material" refers to a product resulting from the enzymatic or metabolic decomposition of an organic substance using a microorganism. For example, the fermented material may comprise a culture itself, which is obtained by culturing a microorganism in a culture medium, or a concentrate, dry product, or lyophilized product of the culture, which is obtained by removing the microorganism therefrom. In addition, in this case, the fermentation broth may comprise the entire fermented material comprising the fermentation product, or may be the fermented material comprising the fermentation product, from which impurities have been removed.

The "microorganism producing the fermentation product" or the "microorganism producing the fermentation product or target product" used in the first step (S100) comprises both a wild-type microorganism and a microorganism that has undergone natural or artificial genetic modification, and may be a microorganism in which a specific mechanism is weakened or strengthened due to insertion of a foreign gene or an activity enhancement or inactivation of an endogenous gene, etc., and it may be a microorganism comprising genetic modification for production of the target protein or a fermentation product.

The microorganism producing the fermentation product of the present disclosure may be a microorganism naturally having the fermentation product-producing ability or a microorganism prepared by providing the fermentation product-producing ability for a parent strain without the fermentation product-producing ability, but is not limited thereto. Specifically, in the present disclosure, the microorganism producing the fermentation product or target product or the microorganism having the fermentation product or target product-producing ability may be a microorganism in which some of the genes involved in the target protein or target product biosynthesis pathway are enhanced or weakened, or some of the genes involved in the target protein or target product degradation pathway are enhanced or weakened. The "enhancement" or "increase" in the fermentation product-producing ability of the microorganism of the present disclosure means that the fermentation product-producing ability of the microorganism of the present disclosure is improved, as compared to microorganisms other than the microorganism of the present disclosure, a parent strain, or an unmodified microorganism. For example, the fermentation product-producing ability of the microorganism of the present disclosure may be improved by about 1% or more, 10% or more, 100% or more, 200% or more, 500% or more, 1000% or more, 1100% or more, 1200% or more, or 1300% or more, and improved by about 1.01 times or more, 2 times or more, 5 times or more, 10 times or more, 11 times or more, 12 times or more, or 13 times or more, as compared to that of other microorganisms, but is not limited thereto. The term "about" comprises all ranges of ±0.5, ±0.4, ±0.3, ±0.2, ±0.1, etc., and comprises all numerical values within the ranges equivalent to or similar to the numerical value following the term "about", but is not limited thereto.

The above-described microorganism used in the first step (S100) may be at least one selected from the group consisting of yeast *Candida famata*, ascomycetes *Eremothecium ashbyii* and *Ashbya Gossypii,* bacteria *Bacillus subtilis* and microorganisms of the *Corynebacterium* sp.

When the microorganism used in the first step (S100) is a microorganism of the *Corynebacterium* sp., the microorganism may be specifically *Corynebacterium glutamicum, Corynebacterium crudilactis, Corynebacterium deserti, Corynebacterium efficiens*, *Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris*, *Corynebacterium crenatum*, or *Corynebacterium flavescens*, and more specifically, *Corynebacterium glutamicum*, but is not limited thereto.

The first step (S100) may further comprise the step of culturing the "microorganism producing the fermentation product". The culture of the microorganism may be performed according to suitable media and culture conditions known in the art. Such a culture process may be easily adjusted and used by those skilled in the art based on the selected strain. Specifically, the culture may be a batch type, continuous type, and fed-batch type, but is not limited thereto. As used herein, the term "medium" refers to a mixture comprising, as main ingredients, nutrient materials required for culturing the microorganism, wherein the medium supplies water essential for survival and growth, nutrient materials, and growth factors, etc. Specifically, as for the media and other culture conditions used for culturing the microorganism of the present disclosure, any medium that is commonly used for culturing microorganisms may be used without particular limitation. However, the microorganism of the present disclosure may be cultured under aerobic conditions in a common medium comprising appropriate carbon sources, nitrogen sources, phosphorus sources, inorganic compounds, fermentation products, and/or vitamins, etc., while controlling the temperature, pH, etc.

In the first step (S100), the capacitive deionization (CDI) process refers to a process that removes ionic substances in the process liquid by adsorbing the ionic substances onto an electrode. The CDI process may be performed by flowing the above-described process liquid through the electrode while applying a potential to the electrode. During this process, the fermentation product comprised in the process liquid passes along the flow path without being adsorbed onto the electrode and is provided to the second step (S200), and the ionic impurities are adsorbed onto the electrode, thereby being removed. CDI is a technology that employs the electrochemical principles of removing ions by adsorbing the ions using an ionic adsorption reaction in an electric double layer of the electrode surface with a high specific surface area. When power is applied to the electrode surface, ions are adsorbed, and when a reverse potential is applied, the ions are desorbed, which regenerates the electrode. Since CDI technology operates at a low potential of about 1.2 V, energy consumption is low, and since it does not use acids, bases, or salts when regenerating the electrode, it is environmentally friendly.

The CDI process performed in the first step (S100) may be occasionally performed by a membrane capacitive deionization device (MCDI), which is an improved form of the general CDI process. The membrane capacitive deionization device (MCDI) is characterized in that an ion exchange membrane is formed on the electrode surface to increase selectivity of the ions to be adsorbed. Therefore, in the present disclosure, when mentioned as the CDI process, it comprises the general CDI method or the improved MCDI method.

Impurities removed through CDI in the first step (S100) may be chloride ions (Cl⁻), phosphate ions (PO₄³⁻), sulfate ions (SO₄²⁻), sodium ions (Na⁺), ammonium ions (NH⁴⁺), potassium ions (K⁺), calcium ions (Ca²⁺), magnesium ions (Mg²⁺), or polar organic substances. Both cations and anions may be removed through the CDI process. Therefore, unlike existing technologies that require both a process of passing through a cation exchange resin and a process of passing through an anion exchange resin to remove both cations and anions, it is possible to remove ions through a single step of injecting the process liquid into the CDI process, thereby improving process efficiency. In addition, since an ion exchange resin is not required, a chemical solvent for desorbing the ions which are adsorbed onto the ion exchange resin and regenerating the resin is unnecessary, thereby producing the fermentation product in an environmentally friendly manner. In addition, as compared to a method of separating impurities by adsorbing the fermentation product onto chromatography, the above-described method required an eluting solution to detach the fermentation product which is adsorbed onto the chromatography, and in this process, the concentration of the fermentation product was diluted by the eluting solution. In addition, chemicals such as caustic or hydrochloric acid, etc. were also required. For this reason, a lot of energy (e.g., steam, etc.) was required to remove a large amount of eluting solution mixed with the fermentation product. In comparison therewith, the method of removing impurities using CDI does not employ the eluting solution, and thus the concentration of the fermentation product in the deionized process liquid may be maintained before and after removing the impurities. Therefore, after the CDI process, the energy required for drying to obtain the fermentation product is low, and the fermentation product may be obtained more economically.

In the first step (S100), after performing the CDI process, only 200 ppm or less of ions, comprising positive/negative ions, may be provided in the deionized process liquid.

The pH of the process liquid in the first step (S100) may range from 3 to 11. Within the acidity range of the process liquid described above, the fermentation product may be electrically neutral without being ionized.

In the first step (S100), the process liquid may be introduced into the CDI process at a rate of 1.3 L/min to 5 L/min. As the flow rate of the process liquid becomes slower, the time during which the electrode and the process liquid meet becomes longer, and thus ions comprised in the process liquid may be more adsorbed and removed. However, when the flow rate of the process liquid becomes slower than 1.3 L/min, the process liquid treatment speed may excessively decrease, which may adversely affect the process yield. On the contrary, when the flow rate of the process liquid becomes faster, the process liquid treatment speed increases, whereas the time during which the process liquid and the electrode meet becomes shorter, and therefore, ions comprised in the process liquid may be relatively less removed. Therefore, by controlling the flow rate of the process liquid at 1.3 L/min to 5 L/min, the ionic impurities comprised in the process liquid may be sufficiently removed while securing the process yield. As the range to achieve the above-described object, the flow rate of the process liquid may be 1.3 L/min to 2.6 L/min or 2.6 L/min to 5.0 L/min. The flow rate of the process liquid may be determined by considering process factors, such as the concentration of ionic impurities comprised in the process liquid, the amount of the process liquid, etc., within the above-mentioned range.

After performing the first step (S100), a step of regenerating the electrode by desorbing ions which are adsorbed onto the electrode during the CDI process may be further performed. The step of regenerating the electrode may be performed by desorbing ions by applying a potential opposite to the potential applied when adsorbing the ions onto the electrode. At this time, a solvent such as water, etc. may be applied into the flow path to remove the desorbed ions.

In the first step (S100), a filtration and decolorization process may be further performed on the fermentation broth. Impurities may be additionally removed through filtration and decolorization. The filtration and decolorization process may be performed by a common method and may be omitted if necessary in order to simplify the process.

Before performing the first step (S100), a step of crystallizing and dissolving the fermentation product comprised in the process liquid may be further performed. Through the crystallization and dissolution of the fermentation product, impurities comprised in the fermentation broth may be primarily removed. However, this process may be omitted if necessary in order to simplify the process.

The deionized process liquid prepared after removing ionic impurities in the first step (S100) is then introduced into the spray-drying process.

In the second step (S200), the deionized process liquid is spray-dried to obtain the fermentation product without re-crystallization of the fermentation product.

Spray-drying is also called spraying drying, and is a method capable of simultaneously completing drying and granulation. It is a method capable of directly drying solutions, emulsions, and suspensions into powder or granule products, thereby eliminating evaporation, grinding, and other processes. After spray-drying, the fermentation product may be dispersed into particles, and most of the moistures may be removed so that the fermentation product in the deionized process liquid may be dried into powder.

Spray-drying is a method of instantly obtaining a dry product of liquid-phase by spraying a liquid in a hot air stream at once, and comprises centrifugal spraying using a rotating disc and pressurized spraying using a pressure nozzle, but is not limited thereto.

During spray-drying, it may be performed by, for example, setting the inlet temperature of the hot air at 120°C to 250°C and the outlet temperature thereof at 30°C to 150°C, preferably 50°C to 100°C, but is not limited thereto.

When the fermentation product is obtained using spray-drying, there is no need for a process of re-crystallizing the deionized process liquid comprising the fermentation product and separating the crystallized fermentation product in order to obtain the fermentation product, as in the existing process. In the case of the existing technology, there has been a problem that many impurities remained in the process liquid even after the deionization process, or the concentration of the fermentation product became low as the fermentation product was diluted by the eluting solution after the deionization process, as previously reviewed. Therefore, in order to obtain a high-purity fermentation product, a process of crystallizing and separating the fermentation product was required. However, when the fermentation product is separated through crystallization, it is apprehended that the fermentation product may be lost during the crystallization and crystal separation process.

In contrast, in the present disclosure, the concentration of the fermentation product is not lowered during the deionization process, but rather, only ionic impurities are removed, and therefore, the concentration of the fermentation product before and after deionization is the same. Accordingly, a high-purity fermentation product may be obtained without crystallizing the fermentation product. In addition, since an eluting solution is not added in the deionization process, the moisture content is relatively low, making it suitable for introduction to the spray-drying process. When the eluting solution is used as in the existing technology, the moisture content in the process liquid increases, and it may be difficult to completely evaporate the moisture through spray-drying.

According to the present disclosure, ionic impurities may be removed without using an ion exchange resin, and a high-purity fermentation product may be obtained at a high yield in an environmentally friendly manner by using the CDI process and the spray-drying process. In addition, according to the present disclosure, since the process of crystallizing the deionized process liquid is not needed, the generation of the solution (mother liquor) remaining after removing the fermentation product may be reduced.

According to the present disclosure, it is possible to secure a high fermentation product recovery rate of 95% or more.

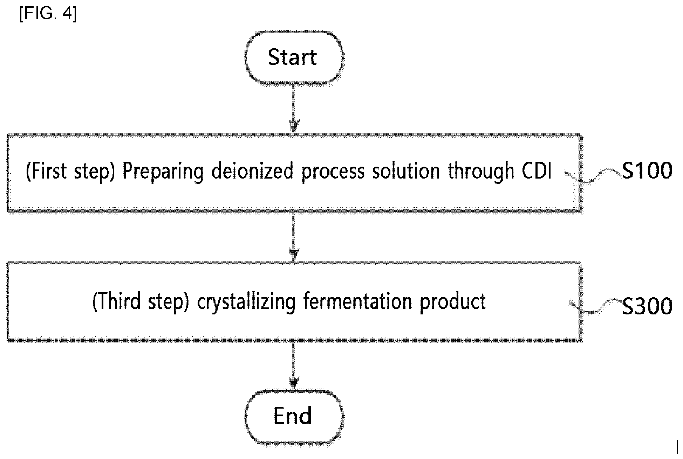

FIG. 4 is a flow chart depicting the method of producing the fermentation product according to the present disclosure.

According to FIG. 4, after performing the first step (S100) of preparing the deionized process liquid via CDI, a third step (S300) of crystallization is performed.

Since the details of the first step (S100) are the same as those discussed above in FIG. 1, they are omitted below to avoid duplication of explanation.

The crystallization of the third step (S300) is a step of obtaining a fermentation product in crystal-form by crystallizing the process liquid from which ionic impurities have been removed. The crystallization of the third step (S300) may be performed by various methods, and there are no limitations on the method. For example, the crystallization of the third step (S300) may be performed by various methods such as adding a coagulant, adjusting pH, cooling, concentrating, etc. In addition, various solid-liquid separation methods, such as separation using a basket, centrifugation, etc., may be performed to separate the crystallized fermentation product.

Therefore, the CDI process exhibits excellent effect in removing ionic impurities without diluting the process liquid as described above, and it does not necessarily have to be used together with the spray-drying process. The CDI process may be combined with various crystallization processes and subsequent processes.

Hereinabove, the process of producing the fermentation product according to one aspect of the present disclosure has been described. Hereinbelow, a device capable of performing the above-described process of producing the fermentation product will be described.

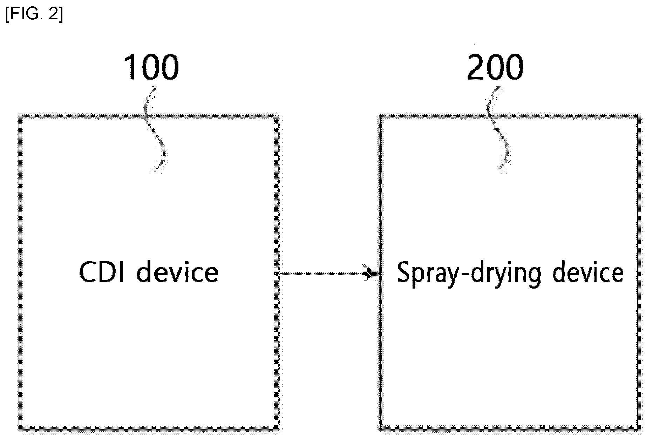

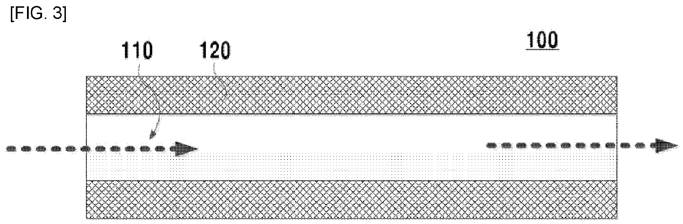

FIG. 2 is a block diagram showing the device for producing the fermentation product according to the present disclosure, and FIG. 3 is a block diagram showing a CDI device in the device for producing the fermentation product according to one embodiment of the present disclosure.

Referring to FIG. 2, a device comprises a capacitive deionization (CDI) device (100) for preparing a deionized process liquid by removing ions from a process liquid comprising the fermentation product; and a spray-drying device (200) for obtaining the fermentation product from the deionized process liquid prepared by the CDI device without re-crystallization.

Referring to FIG. 3, the CDI device (100) may comprise an electrode unit 120 for adsorbing and removing ions comprised in the process liquid when power is applied; and a flow path unit (110) provided such that the process liquid is discharged to the outside of the CDI device (100) after coming into contact with the electrode unit (120).

The flow path unit (110) may be in the form of a straight line extending in one direction as shown in the drawing, but may be implemented in various configurations to increase the time during which the electrode unit (120) and the process liquid meet, if necessary.

The flow path unit (110) may be made of a material with low reactivity so as not to react with the process liquid. Inside the CDI device (100), the flow path unit (110) may be provided alone or may be provided in multiple units in parallel. The number, configuration, size, etc. of the flow path unit (110) may be determined in consideration of the purpose of performing the process, process capacity, etc.

The electrode unit (120) may be connected to a power supply unit so that a potential may be applied thereto. The power supply unit may be provided inside the CDI device (100) or outside of the CDI device (100). The electrode unit (120) is provided adjacent to the flow path unit (110). In some cases, as shown in the drawing, a pair of electrode units (120) may be arranged so as to be each in contact with the flow path units (110). In this case, the pair of electrode units (120) may have potentials in different directions. For example, a potential of the (+) pole may be applied to one electrode, and a potential of the (-) pole may be applied to the other side electrode. Accordingly, anionic impurities and cationic impurities comprised in the process liquid may be all adsorbed onto the electrode units (120), thereby being removed. The direction of the potential applied to the electrode unit (120) may vary depending on the operation of the power supply unit. Accordingly, the electrode unit (120) may adsorb or desorb ionic impurities.

The electrode unit (120) may have a high specific surface area. For example, the electrode unit (120) may comprise a porous surface or a convex-concave structure. Ions may be adsorbed and removed by electrochemical principles of using the adsorption reaction of ions in an electric double layer of the surface of the electrode unit (120) having a high specific surface area.

The spray-drying device (200) may comprise an air heating unit, a spray nozzle, a drying device, a product recovery device, etc. The spray nozzle may be a device that atomizes the process liquid in liquid-state into a desired size of liquid particles, sprays the liquid particles, and disperses the same into a high-temperature gas flow. The solvent in the sprayed liquid particles instantly evaporates in the high-temperature gas flow, and high-purity fermentation product particles may be produced. The fermentation product particles may be moved to the product recovery device by the air flow, and then recovered. The product recovery device may generate an intake airflow inside a drying room to recover the fermentation product particles.

In some cases, the CDI device (100) may be implemented in the form of a membrane capacitive deionization device (MCDI). In this case, an anion exchange resin may be provided at the positive pole of the electrode unit (120) comprised in the CDI device (100), and a cation exchange resin may be provided at the negative pole.

Hereinabove, the device for producing the fermentation product according to one aspect of the present disclosure has been described. The device for producing the fermentation product according to the present disclosure may produce a high-purity fermentation product in an environmentally friendly manner with a high yield by using the CDI device (100) and the spray-drying device (200).

### [Mode for Carrying Out the Invention]

Hereinafter, the present disclosure will be described in more detail by way of exemplary embodiments. However, the following exemplary embodiments are only preferred embodiments for illustrating the present disclosure, and thus are not intended to limit the scope of the present disclosure thereto. Meanwhile, technical matters not described in the present specification may be sufficiently understood and easily implemented by those skilled in the technical field of the present disclosure or similar technical fields.

Hereinabove, a process and a device for producing a fermentation product according to one aspect of the present disclosure have been described. Hereinbelow, it is intended to describe the advantageous effects mentioned in the present disclosure through experimental results of Examples and Comparative Examples.

### Example 1: Analysis of deionization rate and fermentation product recovery rate using CDI

An experiment was conducted using a 100-L scale CDI equipment, and a flow rate was applied at 5 L/min. When feed was applied to the CDI equipment, ion adsorption and desorption process was repeated.

The 100-L scale CDI equipment was used and operated at 5 L/min, and the experiment was performed using, as a feed solution, a mixture of leucine, isoleucine, and valine comprising ions. At this time, the deionization rate was 83% to 88%. The deionization rate was measured by conductivity and concentrations of Na⁺ and SO₄²⁻. The recovery rate of amino acids which are fermentation products in Example 1 was about 98% to 100%. Even though three types of fermentation products such as leucine, isoleucine, and valine were mixed, the fermentation products were recovered at the same level of 98% to 100%. In addition, the concentration of fermentation products in the adsorbent solution was the same as that in the feed, confirming that the fermentation product was not diluted despite the removal of ions. When the fermentation product was not diluted, steam for additional concentrating is not required, and thus steam costs may be reduced.

**[Table 1]**

| Additive | Flow rate, L/min | Process liquid | VAL concentrat ion | ILE concentrat ion | LEU concentr ation | Conductiv ity | Na⁺ concentr ation | SO₄²⁻ concentr ation |
|---|---|---|---|---|---|---|---|---|
| | | | g/L | g/L | g/L | mS | ppm | ppm |
| LEU, ILE, VAL (BCAA) | 5 L/min | Feed | 4.3 | 4.1 | 8.3 | 0.20 | 11 | 29 |
| | | Adsorbing solution | 4.3 | 4.4 | 8.4 | 0.03 | 2 | 3 |
| | | Deionization rate, % | - | - | - | 83.3 | 77.5 | 88.2 |
| | | Fermentation product recovery rate, % | 98.3 | 107.7 | 102 | - | - | - |

### Example 2: Analysis of deionization rate and fermentation product recovery rate according to CDI flow rate

An experiment was conducted by adjusting the flow rate to 1.3 L/min and 2.6 L/min for the 100-L scale CDI equipment. For the process liquid, a solution in which citrulline crystals were dissolved was used. As a result of the experiment, the deionization rate tended to increase from 63% - 66% to 79% - 82% as the flow rate changed from 2.6 L/min to 1.3 L/min. The fermentation product recovery rate was the same at 100% regardless of the change in the flow rate.

Based on these results, the deionization rate is expected to improve as the flow rate is lower, but the hourly processing amount of the process liquid decreases. Thus, it seems that the optimal flow rate should be determined in terms of the hourly processing amount and the deionization rate.

**[Table 2]**

| Additive | Flow rate, L/min | Process liquid | CIT concentration | Conductivity | NH₄⁺ concentration | SO₄²⁻ concentration |
|---|---|---|---|---|---|---|
| | | | g/L | mS | ppm | ppm |
| Citrulline crystal-dissolved solution | 1.3 L/min | Feed | 85.3 | 2.23 | 410 | 1050 |
| | | Adsorbing solution | 89.3 | 0.46 | 90 | 190 |
| | | Deionization rate, % | - | 79.4 | 79.1 | 82.4 |
| | | Fermentation product recovery rate, % | 104.7 | - | - | - |
| | 2.6 L/min | Adsorbing solution | 90.1 | 0.83 | 140 | 350 |
| | | Deionization rate, % | - | 63 | 65.9 | 66.5 |
| | | Fermentation product recovery rate, % | 105.6 | - | - | - |

### Example 3: Operating conditions and product quality during spray-drying after deionization by CDI

Spray-drying was performed using the citrulline-dissolved solution from which ions were removed by CDI in Example 2. During spray-drying, drying was conducted under conditions of an inlet temperature of 200°C and an outlet temperature of 90°C using a nozzle-type spray-dryer.

**[Table 3]**

| Spray-dry operating conditions | | |
|---|---|---|
| Type | Inlet Temp. (°C) | Outlet Temp. (°C) |
| Nozzle | 200 | 90 |

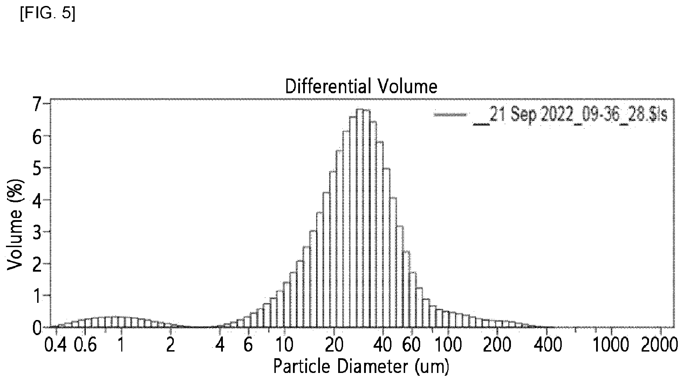

After performing drying under the corresponding conditions, it was confirmed that the moisture content in the powder was less than 0.2%, and the citrulline recovery was achieved at a content of 98.5%, the Cl⁻ content was 0.02% or less, and the NH⁴⁺ content was 0.02% or less, indicating that ion removal in the dry citrulline crystals was possible. At this time, as confirmed in FIG. 5, the median particle size was about 27.1 µm.

### Comparative Example 1: Method of removing anions and separating citrulline using cationic resin

To remove cells from the fermentation broth, pH was adjusted to 4 using sulfuric acid, and cells were separated using membrane separation. Ions in the cell-separated solution were present at 14.6% based on the total solid weight. In this regard, since anions were the predominant ions, ionic impurities were separated using a cationic resin. The pH was adjusted to 1 using sulfuric acid to adsorb citrulline onto the cationic resin by making it cationic. The cell-separated solution of which pH was adjusted to 1 was passed through a cationic resin tower to adsorb citrulline, followed by washing with water to remove impurities other than cations in the cell-separated solution. Thereafter, citrulline was separated by eluting with a 0.1 N sodium hydroxide solution. When citrulline is separated using the cationic resin in this manner, anions are completely removed, and thus 9.8% of impurities, based on the total solid weight, may be separated. However, there are disadvantages in that it is difficult to separate 4.8% of cations, based on the total solid weight, and chemicals such as sulfuric acid, sodium hydroxide, etc. are used. When chemicals are used, it is not desirable in terms of ESG, as well as there is a disadvantage of increasing the material costs for refining due to the use of chemicals.

**[Table 4]**

| Process liquid | | | Cell-separated solution |
|---|---|---|---|
| CIT concentration | | g/L | 78.4 |
| Total solid weight (TS) | | % | 10.3 |
| Purity | | % | 72.9 |
| Ion (g/L) | Cl⁻ | g/L | 0.1 |
| | PO₄³⁻ | | 2.0 |
| | SO₄²⁻ | | 8.0 |
| | Na⁺ | | 0.6 |
| | NH₄⁺ | | 2.3 |
| | K⁺ | | 1.7 |
| | Ca²⁺ | | 0.2 |
| | Mg²⁺ | | 0.1 |
| | Total ion | % | 14.6 |

### Comparative Example 2: Method of removing ions using chromatography resin

It was observed that when the feed was purified using chromatography, the deionization rate was 98%, which means that ions may be removed at a high level. However, in terms of citrulline concentration, the citrulline concentration, which was 110 g/L when transferred to the process liquid after ion removal from the feed, was diluted to 28.9 g/L, which was about 3.8 times lower. Therefore, even though chromatography is efficient in ion removal, there has been a problem that steam costs are additionally required in the final production process as the fermentation product in the process liquid is diluted.

**[Table 5]**

| Process liquid | | Feed | Extract (deionized process liquid) | Raffinate 1 | Raffinate 2 |
|---|---|---|---|---|---|
| Citrulline concentration | g/L | 110.5 | 28.9 | 1.5 | 7.8 |
| Conductivity | mS | 15.78 | 0.23 | 4.73 | 8.27 |
| Balance | % | - | 73.5 | 1.4 | 19.8 |
| Total balance | % | - | 94.7 | | |
| Recovery rate | % | - | 73.5 | | |

### Comparative Example 3: Method of removing ions using crystallization

Table 6 shows the data when ions were removed from the feed through the crystallization process and only citrulline crystals were obtained. At this time, a deionized process liquid (SMB Extract) in which ions were removed through continuous chromatography was used as a feed, and cooling crystallization was performed at 35°C. At this time, the deionization rate of citrulline crystals in the feed was 90.9%, and the recovery rate of crystals was 80.32%. Since it is less than 0.02% which is a product specification standard for citrulline, citrulline crystals satisfying all the product specification may be produced. However, when ions are removed through crystallization, the recovery rate is 80%, resulting in 20% loss of citrulline, and a crystallization mother liquor is generated when separating the crystals. This mother liquor must be recycled back into the upstream process or treated as wastewater, which poses a disadvantage in terms of the environment and rise in costs due to wastewater treatment.

**[Table 6]**

| Process liquid | | | Feed | Citrulline crystallization mother liquor | Citrulline crystal |
|---|---|---|---|---|---|
| Citrulline concentration | | g/L | 80.4 | 147.65 | - |
| Citrulline content | | % | - | - | 98.88 |
| Purity | | % | 94.5 | 72.7 | 99.3 |
| Recovery rate | | % | - | - | 80.32 |
| Ion (g/L) | Cl⁻ | g/L | 0.01 | 0.26 | 0.03 |
| | PO₄³⁻ | | 0.02 | 0.34 | 0.01 |
| | SO₄²⁻ | | 0.05 | 0.47 | 0.01 |
| | Na⁺ | | 0.02 | 0.26 | 0.02 |
| | NH₄⁺ | | 0.94 | 1.42 | 0.03 |
| | K⁺ | | 0.09 | 0.72 | 0.02 |
| | Ca²⁺ | | 0.01 | 0.08 | 0.01 |
| | Mg²⁺ | | 0.00 | 0.01 | 0.00 |
| Total ion | | % | 0.11 | 0.35 | 0.01 |

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

## Claims

1. A method of producing a fermentation product, the method comprising:
a first step of preparing a deionized process liquid by removing ions from a process liquid comprising the fermentation product via capacitive deionization (CDI); and
a second step of spray-drying the deionized process liquid to obtain the fermentation product without re-crystallization of the fermentation product.

2. The method of claim 1, wherein the fermentation product comprises one or more selected from the group consisting of leucine, isoleucine, citrulline, and valine.

3. The method of claim 1, wherein in the first step, the process liquid is introduced into the CDI process at a rate of 1.3 L/min to 5 L/min.

4. The method of claim 1, further comprising the step of regenerating an electrode by desorbing the ions which are adsorbed onto the electrode during the CDI process, after performing the first step.

5. The method of claim 1, further comprising the step of preparing the process liquid comprising the fermentation product by fermentation utilizing microbial cells, before performing the first step.

6. The method of claim 5, further comprising the step of crystallizing and dissolving the fermentation product comprised in the process liquid, before performing the first step.

7. A device for producing a fermentation product, the device comprising:
a capacitive deionization (CDI) device for preparing a deionized process liquid by removing ions from a process liquid comprising the fermentation product; and
a spray-drying device for obtaining the fermentation product from the deionized process liquid prepared by the CDI device without re-crystallization.

8. The device of claim 7, wherein the CDI device comprises an electrode unit for adsorbing and removing ions comprised in the process liquid, when power is applied; and
a flow path unit provided such that the process liquid is discharged to the outside of the CDI device after coming into contact with the electrode unit.

9. A method of producing a fermentation product, the method comprising:
a first step of preparing a deionized process liquid by removing ions from a process liquid comprising the fermentation product via capacitive deionization (CDI); and
a third step of obtaining a fermentation product in crystal-form from the deionized process liquid.
